# EUROPEAN PATENT APPLICATION

(11) **EP 4 838 204 A1**
(43) Date of publication of application: **11.11.2026**
(21) Application number: 25917970.3
(22) Date of filing: 25.11.2025
(51) Int. Cl.: G01N 17/00

(54) **METHOD FOR EVALUATING IRRADIATION EMBRITTLEMENT OF METAL MATERIAL ON BASIS OF SURFACE THERMAL CONDUCTIVITY**

(30) Priority: 07.05.2025 CN 202510584878
(71) Applicant: Suzhou Nuclear Power Research Institute Co., Ltd., Suzhou, Jiangsu 215004 (CN)
(72) Inventor: XU, Chaoliang, Suzhou, Jiangsu 215004 (CN); LIU, Xiangbing, Suzhou, Jiangsu 215004 (CN); LI, Yuanfei, Suzhou, Jiangsu 215004 (CN); JIA, Wenqing, Suzhou, Jiangsu 215004 (CN); QUAN, Qiwei, Suzhou, Jiangsu 215004 (CN); YIN, Jian, Suzhou, Jiangsu 215004 (CN); JIN, Xiao, Suzhou, Jiangsu 215004 (CN); WU, Huanchun, Suzhou, Jiangsu 215004 (CN); QIAN, Wangjie, Suzhou, Jiangsu 215004 (CN)
(74) Representative: Westphal, Mussgnug & Partner, Patentanwälte mbB
(86) International application number: PCT/CN2025/137586
(87) International publication number: WO 2026/149048

(57) **Abstract**

Disclosed in the present invention is a method for evaluating the irradiation embrittlement of a metal material on the basis of surface thermal conductivity. The method comprises the following steps: S1, irradiating a metal sample to be tested, and establishing an irradiation damage dose - damage depth curve, so as to obtain the maximum irradiation damage depth; S2. measuring the nano-hardness of unirradiated metal specimens and irradiated metal specimens having different irradiation damage doses; S3, determining a test laser frequency, and measuring the surface thermal conductivity of the unirradiated metal specimens and the irradiated metal specimens having different irradiation damage doses; S4, establishing a nano-hardness - surface thermal conductivity curve; S5, on the basis of the test laser frequency, measuring the surface thermal conductivity of the irradiated metal sample to be tested, and determining the nano-hardness of said irradiated metal sample; and S6, calculating the ductile-to-brittle transition temperature increment of said irradiated metal sample. By means of non-destructive measurement of the surface thermal conductivity of the metal material, the present invention effectively evaluates the irradiation embrittlement level of the metal material, and has the advantages of being non-destructive and capable of remote and real-time measurement.

## Description

### TECHNICAL FIELD

The present invention relates to the technical field of metal material evaluation technology, and particularly relates to a method for evaluating irradiation embrittlement of a metal material on basis of surface thermal conductivity.

### BACKGROUND TECHNOLOGY

Metal materials operating in irradiated environments (such as reactors and accelerators) often exhibit irradiation embrittlement due to the formation of irradiation defects, which leads to reduced toughness of metal components and increases the likelihood of fracture failure. Evaluating irradiation embrittlement of metal materials is a crucial requirement for evaluating the service performance of components. Irradiation embrittlement is typically characterized by the ductile-to-brittle transition temperature increment ΔT, reflecting the degree of irradiation embrittlement and serving as a key indicator characterizing irradiation embrittlement, with significant engineering implications. For example, in nuclear power plant reactor pressure vessels, if the ΔT value after irradiation is large, it may exceed the limits specified in technical specifications, causing the safety of the corresponding components to fail to meet standard requirements.

Typically, the ΔT value of irradiated a metal material is obtained using the Charpy impact test. However, there are significant limitations to using the Charpy impact test to assess irradiation embrittlement: 1) The Charpy impact test requires a large sample size and is a destructive test, which may lead to insufficient evaluation samples when test materials are limited. 2) The Charpy impact test is an offline test. In specific engineering evaluations, the component being evaluated may not be able to be cut and sampled, and can only be tested using simulated samples in similar service environments, which may result in the evaluation results not accurately reflecting the actual engineering component. 3) In some special applications (such as irradiated environments, vacuum environments, liquid environments, etc.), it is not possible to continuously monitor the level of irradiation embrittlement of a metal material remotely.

### SUMMARY OF THE INVENTION

The technical problem to be solved by the present invention is to provide a method for evaluating irradiation embrittlement of metal material on basis of surface thermal conductivity.

The technical solution adopted by the present invention to solve the above technical problem is to provide a method for evaluating irradiation embrittlement of a metal material on basis of surface thermal conductivity, including the following steps:
S1. Irradiating metal sample to be tested, establishing the irradiation damage amount-damage depth curve, and obtaining the maximum irradiation damage depth;
S2. Measuring the nanohardness of an unirradiated metal sample and irradiated metal samples with different irradiation damage amounts;
S3. Determining test laser frequency and measuring the surface thermal conductivity of the unirradiated metal sample and the irradiated metal samples with different irradiation damage amounts;
S4. According to the measurement results of steps S2 and S3, establishing the nanohardness-surface thermal conductivity curve;
S5. Based on the test laser frequency, measuring the surface thermal conductivity of the metal sample to be tested after irradiation, and determining the nanohardness of the metal sample to be tested after irradiation according to the nanohardness-surface thermal conductivity curve;
S6. Based on the nanohardness of the metal sample to be tested after irradiation and the nanohardness of the unirradiated metal sample, calculating the ductile-to-brittle transition temperature increment of the metal sample to be tested after irradiation to evaluate the irradiation embrittlement level of the metal material.

In some embodiments, in step S1, the irradiation is proton irradiation, heavy ion irradiation, or neutron irradiation, and the irradiation energy is 220 keV to 260 keV; and/or,
Based on the elements with a mass percentage ≥ 5% in the metal sample to be tested, calculating the irradiation damage amount-damage depth curve of the metal sample to be tested after irradiation.

In some embodiments, the maximum irradiation damage depth dₘₐₓ and the indentation depth dₙ for measuring nanohardness satisfy the following relationship: dₙ≥0.2dₘₐₓ.

In some embodiments, step S3 includes the following sub-steps:
S3.1. Performing surface coating to a standard sample, the unirradiated metal sample, and the irradiated metal samples with different irradiation damage amounts;
S3.2. Selecting a first laser frequency and a second laser frequency, performing a calibration test by adopting a coated standard sample, and performing an applicability test by adopting an coated unirradiated metal sample;
S3.3. Determining a test depth of the coated unirradiated metal sample and an coated irradiated metal sample;
S3.4. Calculating the test laser frequency based on the test depth;
S3.5. Based on the test laser frequency, measuring the surface thermal conductivity of the unirradiated metal sample and the irradiated metal samples.

In some embodiments, in step S3.1, the standard sample is aluminum oxide; and/or, the coating thickness is 70 nm to 90 nm; and/or, the composition of the coating is aluminum or gold.

In some embodiments, in step S3.2, the first laser frequency and the second laser frequency are both 0.1MHz to 10MHz.

In some embodiments, in step S3.2, in the calibration test, performing a surface thermal conductivity test to the coated standard sample to obtain the thermal conductivity k_{b1} of the coated standard sample at the first laser frequency and the thermal conductivity k_{b2} of the coated standard sample at the second laser frequency, and judging whether the following relationship is satisfied: k_{b1}=k_{b2}=theoretical thermal conductivity value of the standard sample; if so, performing the applicability test; and/or, in the applicability test, performing a surface thermal conductivity test to the coated unirradiated metal sample to obtain the thermal conductivity kᵤ₁ of the coated unirradiated metal sample at the first laser frequency and the thermal conductivity kᵤ₂ of the coated unirradiated metal sample at the second laser frequency, and judging whether the following relationship is satisfied: kᵤ₁=kᵤ₂; if so, performing the next step.

In some embodiments, in step S3.3, the test depth dₖ and the maximum irradiation damage depth dₘₐₓ satisfy the following relationship: 0.2dₘₐₓ ≤dₖ≤0.9dₘₐₓ.

In some embodiments, in step S3.4, calculating the test laser frequency f₀ by equation (1):
${f}_{0} = \sqrt{\frac{{k}_{u 1}}{{πCd}_{k}}}$ (1)

Wherein, kᵤ₁ represents the thermal conductivity of the coated unirradiated metal sample at the first laser frequency, C represents the volumetric heat capacity of the coated unirradiated metal sample, and dₖ represents the test depth.

In some embodiments, in step S6, calculating the ductile-to-brittle transition temperature increment ΔT of the metal sample to be tested after irradiation by equation (2):
$ΔT = 178.1 \times \left({H}_{p}-{H}_{0}\right)$ (2)

Wherein, Hₚ represents the nanohardness of the metal sample to be tested after irradiation, and H₀ represents the nanohardness of the unirradiated metal sample.

The present invention has the following beneficial effects: The method for evaluating irradiation embrittlement of a metal material of the present invention can effectively evaluate the irradiation embrittlement level of a metal material by performing non-destructive measuring the surface thermal conductivity of a metal material. The method for evaluating irradiation embrittlement of the present invention has the following advantages: 1) It does not cause damage to the metal material; 2) It can evaluate irradiation embrittlement level remotely or across transparent media; 3) It can evaluate irradiation embrittlement level in real time.

### BRIEF DESCRIPTION OF THE DRAWINGS

The present invention will be further described below in conjunction with accompanying drawings and embodiments. In the drawings,
FIG. 1 is the irradiation damage amount-damage depth curve of Embodiment 1 of the present invention; and
FIG. 2 is the nanohardness-surface thermal conductivity curve of Embodiment 1 of the present invention.

### DETAILED DESCRIPTION OF THE EMBODIMENTS

To gain a thorough understanding of the technical features, purposes and effects of the invention, specific details are given below in combination with the embodiments. The embodiments are only used to explain the present invention and do not constitute a limitation of the protection scope of the present invention.

The present invention provides a method for evaluating irradiation embrittlement of a metal material on basis of surface thermal conductivity, the metal material can be reactor pressure vessel (RPV) steel. The method for evaluating irradiation embrittlement includes the following steps:
S1. Irradiating metal sample to be tested, establishing the irradiation damage amount-damage depth curve, and obtaining the maximum irradiation damage depth.

In step S1, the irradiation is proton irradiation, heavy ion irradiation, or neutron irradiation, and the irradiation energy can be 220keV~260keV, such as 220keV, 230keV, 240keV, 250keV, or 260keV. In other embodiments, the irradiation can also be performed by adopting protons, heavy ions, or neutrons with other irradiation energy values. The specific irradiation conditions can be selected according to actual needs. Based on the elements with a mass percentage ≥5% in the metal sample to be tested, the irradiation damage amount-damage depth curve of the metal sample to be tested after irradiation is calculated. The displacement threshold energies of each metal element are as follows: Fe (iron), Cr (chromium), Ni (nickel), Mn (manganese), Co (cobalt), and Zr (zirconium) have a displacement threshold energy E_{d}=40 eV; Al (aluminum) and Pb (lead) have a displacement threshold energy E_{d}=25 eV; Cu (copper) and Ti (titanium) have a displacement threshold energy E_{d}=30 eV; Mo (molybdenum) and Nb (niobium) have a displacement threshold energy E_{d}=60 eV; and W (tungsten) has a displacement threshold energy E_{d}=90 eV. Furthermore, based on the irradiation damage amount-damage depth curve, the maximum irradiation damage depth dₘₐₓ corresponding to the maximum irradiation damage amount in the curve is determined. If the irradiation damage amount distribution in the curve is uniform, then the maximum irradiation damage depth dₘₐₓ is assigned the value dₘₐₓ=1 µm.

S2. Measuring the nanohardness of an unirradiated metal sample and irradiated metal samples with different irradiation damage amounts.

In step S2, nanohardness is measured by a nanoindenter. During the measurement, the maximum irradiation damage depth dₘₐₓ and the indentation depth dₙ of the nanoindentation satisfy the following relationship: dₙ ≥0.2dₘₐₓ. Different irradiation damage amounts include at least three, such as three, four, or five, with irradiation damage amounts such as 0.1 dpa, 0.3 dpa, 0.7 dpa, 1.0 dpa, and 1.6 dpa. The nanohardness H₀ of the unirradiated metal sample and the nanohardness Hᵢ₁, Hᵢ₂, and Hᵢ₃ of the irradiated metal samples with at least three irradiation damage amounts are obtained through measurement.

S3. Determining test laser frequency and measuring the surface thermal conductivity of the unirradiated metal sample and the irradiated metal samples with different irradiation damage amounts. Specifically, step S3 includes the following sub-steps:
S3.1. Performing surface coating to a standard sample, the unirradiated metal sample, and the irradiated metal samples with different irradiation damage amounts.

In step S3.1, the standard sample can be aluminum oxide (Al₂O₃), which has high stability and consistent surface thermal conductivity. The coating thickness can be 70nm~90nm, such as 70nm, 75nm, 80nm, 85nm, or 90nm. The composition of the coating can be aluminum (Al) or gold (Au). Surface coating can be performed by methods such as magnetron sputtering or arc ion plating. Understandably, surface coating of metal materials belongs to prior art, and process parameters of it can be selected according to actual process conditions, without specific limitations here.

S3.2. Selecting a first laser frequency and a second laser frequency, performing a calibration test by adopting a coated standard sample, and performing an applicability test by adopting an coated unirradiated metal sample.

In step S3.2, the first laser frequency f₁ and the second laser frequency f₂ are both selected within the range of 0.1MHz to 10MHz, such as 0.1MHz, 1MHz, 3MHz, 7MHz, or 10MHz. The first laser frequency f₁ and the second laser frequency f₂ are not equal, preferably, f₁ = 1.82 MHz and f₂ = 9.70 MHz.

The calibration test is used to verify the calibration of the adjusted thermal conductivity testing equipment. Specifically, based on the first laser frequency and the second laser frequency, the surface thermal conductivity test to the coated standard sample is performed to obtain the thermal conductivity k_{b1} of the coated standard sample at the first laser frequency and the thermal conductivity k_{b2} of the coated standard sample at the second laser frequency. It is then judged whether the following relationship is satisfied: k_{b1}=k_{b2}=theoretical thermal conductivity value of the standard sample. If so, it indicates that the thermal conductivity testing equipment has passed calibration and can proceed to the applicability test; if not, the thermal conductivity testing equipment needs to be readjusted. The theoretical thermal conductivity value of aluminum oxide is 36 W/mK.

In the applicability test, the thermal conductivity testing equipment passed calibration is used to verify whether the metal material is suitable for the method for evaluating irradiation embrittlement of the present invention. Specifically, based on the first laser frequency and the second laser frequency, the surface thermal conductivity test to the coated unirradiated metal sample is performed to obtain the thermal conductivity kᵤ₁ of the coated unirradiated metal sample at the first laser frequency and the thermal conductivity kᵤ₂ of the coated unirradiated metal sample at the second laser frequency. It is then judged whether the following relationship is satisfied: kᵤ₁ = kᵤ₂. If so, it indicates that the thickness direction of the metal sample is independent of the laser frequency, and can proceed to the next step; if not, it indicates that the metal material is not suitable for the method for evaluating irradiation embrittlement of the present invention, and the test should be stopped.

S3.3. Determining a test depth of the coated unirradiated metal sample and an coated irradiated metal sample.

In step S3.3, the test depth dₖ and the maximum irradiation damage depth dₘₐₓ satisfy the following relationship: 0.2 dₘₐₓ≤dₖ ≤0.9 dₘₐₓ.

S3.4. Calculating the test laser frequency based on the test depth.

In step S3.4, the test laser frequency f₀ is calculated by equation (1):
${f}_{0} = \sqrt{\frac{{k}_{u 1}}{{πCd}_{k}}}$ (1)

Wherein, kᵤ₁ represents the thermal conductivity of the coated unirradiated metal sample at the first laser frequency in step 3.2, C represents the volumetric heat capacity of the coated unirradiated metal sample, and dₖ represents the test depth in step 3.3.

S3.5. Based on the test laser frequency f₀ in step 3.4, measuring the surface thermal conductivity of the unirradiated metal sample and the irradiated metal samples with different irradiation damage amounts.

S4. According to the nanohardness measured in step S2 and the surface thermal conductivity measured in step S3, establishing the nanohardness-surface thermal conductivity curve by adopting least squares method.

S5. Based on the test laser frequency f₀ in step S3.4, measuring the surface thermal conductivity Kₚ of the metal sample to be tested after irradiation, and determining the nanohardness Hₚ of the metal sample to be tested after irradiation according to the nanohardness-surface thermal conductivity curve in step S4.

S6. Based on the nanohardness Hₚ of the metal sample to be tested after irradiation in step S5 and the nanohardness H₀ of the unirradiated metal sample in step S2, calculating the ductile-to-brittle transition temperature increment of the metal sample to be tested after irradiation to evaluate the irradiation embrittlement level of the metal material.

In step S6, the ductile-to-brittle transition temperature increment ΔT of the metal sample to be tested after irradiation is calculated by equation (2):
$ΔT = 178.1 \times \left({H}_{p}-{H}_{0}\right)$ (2)

Wherein, Hₚ represents the nanohardness of the metal sample to be tested after irradiation, and H₀ represents the nanohardness of the unirradiated metal sample.

The present invention provides a novel method for evaluating irradiation embrittlement of a metal material. By performing non-destructive measuring the surface thermal conductivity of the metal material, the irradiation embrittlement level of the metal material can be effectively evaluated. The method for evaluating irradiation embrittlement of the present invention has the following advantages: 1) The evaluation method does not cause damage to the metal material, making it a non-destructive evaluation technology; 2) The irradiation embrittlement level of the metal material can be evaluated remotely or across transparent media; 3) The irradiation embrittlement level of the metal material can be evaluated in real time.

The following is an illustration through specific embodiment:

### Embodiment 1

The method for evaluating irradiation embrittlement of a metal material on basis of surface thermal conductivity of the present invention is adopted to evaluate the irradiation embrittlement of RPV steel. The method for evaluating includes the following steps:
S1. Irradiating metal sample to be tested, establishing the irradiation damage amount-damage depth curve, and obtaining the maximum irradiation damage depth. The metal sample to be tested is RPV steel.

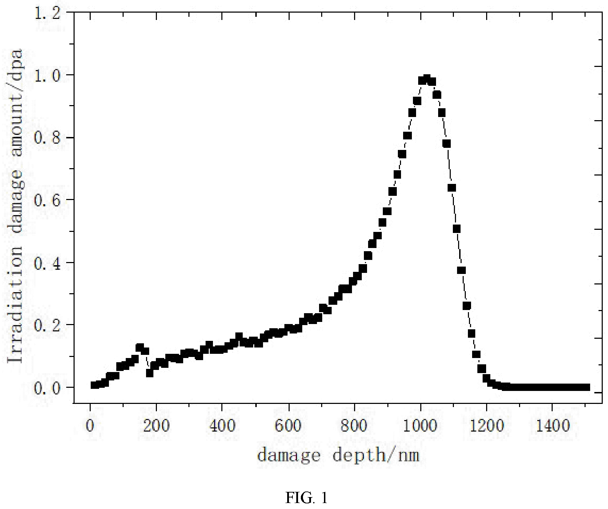

In step S1, the irradiation is 240 keV proton irradiation. Since the Fe content in the RPV steel is ≥95%, only Fe is considered in the calculation, and Fe has a displacement threshold energy E_{d} = 40 eV. The irradiation damage amount-damage depth curve is shown in FIG. 1, and the maximum irradiation damage depth corresponding to the maximum irradiation damage amount is dₘₐₓ=1000 nm.

S2. Measuring the nanohardness of an unirradiated metal sample and irradiated metal samples with different irradiation damage amounts.

In step S2, nanohardness is measured by a nanoindenter. During the measurement, the indentation depth of the nanoindentation is dₙ=0.2dₘₐₓ=200nm. Different irradiation damage amounts includes 0.3dpa, 1.0dpa, and 1.6dpa. The nanohardness H₀ of the unirradiated metal sample and the nanohardness Hᵢ₁, Hᵢ₂, and Hᵢ₃ of the irradiated metal samples with different irradiation damage amounts are obtained. The measurement results are shown in Table 1.

**Table 1. Nanohardness Measurement Results**

| | H₀ | Hᵢ₁ | Hᵢ₂ | Hᵢ₃ |
|---|---|---|---|---|
| Nanohardness/GPa | 2.74 | 4.12 | 4.57 | 4.78 |
| Error/GPa | 0.15 | 0.26 | 0.21 | 0.31 |
| Irradiation damage amount/dpa | 0 | 0.3 | 1.0 | 1.6 |

S3. Determining test laser frequency and measuring the surface thermal conductivity of the unirradiated metal sample and the irradiated metal samples with different irradiation damage amounts. Specifically, step S3 includes the following sub-steps:
S3.1. Performing surface coating to a standard sample, the unirradiated metal sample, and the irradiated metal samples with different irradiation damage amounts. The metal samples are RPV steel samples.

In step S3.1, the standard sample is Al₂O₃, the coating thickness is 80 nm, and the composition of the coating is Al. The surface coating is performing by magnetron sputtering, the specific process parameters can be selected according to actual needs and are not limited.

S3.2. Selecting a first laser frequency and a second laser frequency, performing a calibration test by adopting a coated standard sample, and performing an applicability test by adopting an coated unirradiated metal sample.

In step S3.2, the first laser frequency f₁ = 1.82 MHz and the second laser frequency f₁ = 9.70 MHz.

In the calibration test, based on the first laser frequency f₁ and the second laser frequency f₂, the surface thermal conductivity test to the coated standard sample is performed to obtain the thermal conductivity k_{b1} of the coated standard sample at the first laser frequency and the thermal conductivity k_{b2} of the coated standard sample at the second laser frequency. k_{b1}=k_{b2}=theoretical thermal conductivity value of Al₂O₃ = 36W/mK, indicating that the calibration of the thermal conductivity testing equipment is successful.

In the applicability test, based on the first laser frequency f₁ and the second laser frequency f₂, the surface thermal conductivity test to the coated unirradiated metal sample is performed to obtain the thermal conductivity kᵤ₁ of the coated unirradiated metal sample at the first laser frequency and the thermal conductivity kᵤ₂ of the coated unirradiated metal sample at the second laser frequency. kᵤ₁= kᵤ₂=39.8W/mK, indicating that the thickness direction of the metal sample is independent of the laser frequency.

S3.3. Determining a test depth of the coated unirradiated metal sample and an coated irradiated metal sample.

In step S3.3, the test depth is dₖ=0.88 dₘₐₓ=880nm.

S3.4. Calculating the test laser frequency based on the test depth.

In step S3.4, the test laser frequency f₀ is calculated by equation (1):
${f}_{0} = \sqrt{\frac{{k}_{u 1}}{{πCd}_{k}}}$ (1)

Wherein, kᵤ₁ represents the thermal conductivity of the coated unirradiated metal sample at the first laser frequency in step 3.2, C represents the volumetric heat capacity of the coated unirradiated metal sample, and dₖ represents the test depth in step 3.3.

Based on kᵤ₁=39.8 W/mK, C=4×10⁶J/(m³K), and dₖ=880nm, it is calculated that the test laser frequency f₀=4.12MHz.

S3.5. Based on the test laser frequency f₀ in step 3.4, measuring the surface thermal conductivity of the unirradiated metal sample and the irradiated metal samples with different irradiation damage amounts to obtain the surface thermal conductivity k₀ of the unirradiated metal sample, and the surface thermal conductivity kᵢ₁, kᵢ₂, and kᵢ₃ of the irradiated metal samples with different irradiation damage amounts (0.3 dpa, 1.0 dpa, and 1.6 dpa). The measurement results are shown in Table 2.

**Table 2 Surface Thermal Conductivity Measurement Results [0087]**

| | k₀ | kᵢ₁ | kᵢ₂ | kᵢ₃ |
|---|---|---|---|---|
| Surface thermal conductivity, W/mK | 39.8 | 35.0 | 34.7 | 31.9 |
| Error, W/mK | 0.4 | 0.8 | 1.4 | 1.8 |
| Irradiation damage amount, dpa | 0 | 0.3 | 1.0 | 1.6 |

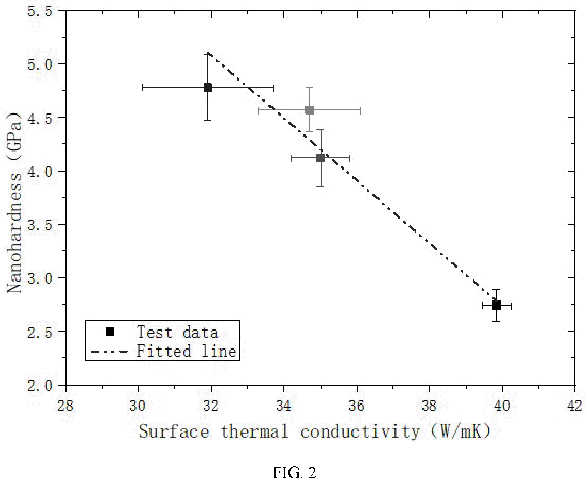

S4. According to the nanohardness measured in step S2 and the surface thermal conductivity measured in step S3, establishing the nanohardness-surface thermal conductivity curve (H-k curve) as shown in FIG. 2 by adopting least squares method. The curve formula is H=-0.29327k+14.46, R²=0.9359.

S5. Based on the test laser frequency f₀ in step S3.4, measuring the surface thermal conductivity Kₚ of the metal sample to be tested after irradiation, and determining the nanohardness Hₚ of the metal sample to be tested after irradiation according to the nanohardness-surface thermal conductivity curve in step S4. Kₚ = 36 W/mK, and Hₚ = 3.90 GPa.

S6. Based on the nanohardness Hₚ of the metal sample to be tested after irradiation in step S5 and the nanohardness H₀ of the unirradiated metal sample in step S2, calculating the ductile-to-brittle transition temperature increment of the metal sample to be tested after irradiation to evaluate the irradiation embrittlement level of the metal material.

In step S6, the ductile-to-brittle transition temperature increment ΔT of the metal sample to be tested after irradiation is calculated by equation (2):
$ΔT = 178.1 \times \left({H}_{p}-{H}_{0}\right)$ (2)

Wherein, Hₚ represents the nanohardness of the metal sample to be tested after irradiation, and H₀ represents the nanohardness of the unirradiated metal sample.

Based on Hₚ=3.90 GPa and H₀=2.74 GPa, it is calculated that the ductile-to-brittle transition temperature increment of the metal sample after irradiation is ΔT = 206.6°C.

Understandably, the above embodiments merely illustrate preferred embodiments of the present invention and are described in a relatively specific and detailed manner, but they should not be construed as limiting the scope of the present invention. It should be noted that those skilled in the art may, without departing from the spirit of the invention, freely combine the above technical features and make various modifications and improvements, all of which fall within the scope of protection of the present invention. Therefore, any equivalent changes and modifications made within the scope of the claims of the present invention should fall within the scope of the claims.

## Claims

1. A method for evaluating irradiation embrittlement of a metal material on basis of surface thermal conductivity, comprising the following steps:
S1. Irradiating metal sample to be tested, establishing the irradiation damage amount-damage depth curve, and obtaining the maximum irradiation damage depth;
S2. Measuring the nanohardness of an unirradiated metal sample and irradiated metal samples with different irradiation damage amounts;
S3. Determining test laser frequency and measuring the surface thermal conductivity of the unirradiated metal sample and the irradiated metal samples with different irradiation damage amounts;
S4. According to the measurement results of steps S2 and S3, establishing the nanohardness-surface thermal conductivity curve;
S5. Based on the test laser frequency, measuring the surface thermal conductivity of the metal sample to be tested after irradiation, and determining the nanohardness of the metal sample to be tested after irradiation according to the nanohardness-surface thermal conductivity curve;
S6. Based on the nanohardness of the metal sample to be tested after irradiation and the nanohardness of the unirradiated metal sample, calculating the ductile-to-brittle transition temperature increment of the metal sample to be tested after irradiation to evaluate the irradiation embrittlement level of the metal material.

2. The method for evaluating irradiation embrittlement of the metal material on basis of surface thermal conductivity according to claim 1, wherein in step S1, the irradiation is proton irradiation, heavy ion irradiation, or neutron irradiation, and the irradiation energy is 220 keV to 260 keV; and/or,
based on the elements with a mass percentage ≥ 5% in the metal sample to be tested, calculating the irradiation damage amount-damage depth curve of the metal sample to be tested after irradiation.

3. The method for evaluating irradiation embrittlement of the metal material on basis of surface thermal conductivity according to claim 1, the maximum irradiation damage depth dₘₐₓ and the indentation depth dₙ for measuring nanohardness satisfy the following relationship: dₙ≥0.2dₘₐₓ.

4. The method for evaluating irradiation embrittlement of the metal material on basis of surface thermal conductivity according to claim 1, step S3 comprises the following sub-steps:
S3.1. Performing surface coating to a standard sample, the unirradiated metal sample, and the irradiated metal samples with different irradiation damage amounts;
S3.2. Selecting a first laser frequency and a second laser frequency, performing a calibration test by adopting a coated standard sample, and performing an applicability test by adopting an coated unirradiated metal sample;
S3.3. Determining a test depth of the coated unirradiated metal sample and an coated irradiated metal sample;
S3.4. Calculating the test laser frequency based on the test depth;
S3.5. Based on the test laser frequency, measuring the surface thermal conductivity of the unirradiated metal sample and the irradiated metal samples.

5. The method for evaluating irradiation embrittlement of the metal material on basis of surface thermal conductivity according to claim 4, wherein in step S3.1, the standard sample is aluminum oxide; and/or, the coating thickness is 70 nm to 90 nm; and/or, the composition of the coating is aluminum or gold.

6. The method for evaluating irradiation embrittlement of the metal material on basis of surface thermal conductivity according to claim 4, wherein in step S3.2, the first laser frequency and the second laser frequency are both 0.1MHz to 10MHz.

7. The method for evaluating irradiation embrittlement of the metal material on basis of surface thermal conductivity according to claim 4 or 6, wherein in step S3.2, in the calibration test, performing a surface thermal conductivity test to the coated standard sample to obtain the thermal conductivity k_{b1} of the coated standard sample at the first laser frequency and the thermal conductivity k_{b2} of the coated standard sample at the second laser frequency, and judging whether the following relationship is satisfied: k_{b1}=k_{b2}=theoretical thermal conductivity value of the standard sample; if so, performing the applicability test; and/or,
in the applicability test, performing a surface thermal conductivity test to the coated unirradiated metal sample to obtain the thermal conductivity kᵤ₁ of the coated unirradiated metal sample at the first laser frequency and the thermal conductivity kᵤ₂ of the coated unirradiated metal sample at the second laser frequency, and judging whether the following relationship is satisfied: kᵤ₁=kᵤ₂; if so, performing the next step.

8. The method for evaluating irradiation embrittlement of the metal material on basis of surface thermal conductivity according to claim 4, wherein in step S3.3, the test depth dₖ and the maximum irradiation damage depth dₘₐₓ satisfy the following relationship: 0.2dₘₐₓ ≤dₖ≤0.9dₘₐₓ.

9. The method for evaluating irradiation embrittlement of the metal material on basis of surface thermal conductivity according to claim 7, wherein in step S3.4, calculating the test laser frequency f₀ by equation (1): ${f}_{0} = \sqrt{\frac{{k}_{u 1}}{{πCd}_{k}}}$ (1) wherein, kᵤ₁ represents the thermal conductivity of the coated unirradiated metal sample at the first laser frequency, C represents the volumetric heat capacity of the coated unirradiated metal sample, and dₖ represents the test depth.

10. The method for evaluating irradiation embrittlement of the metal material on basis of surface thermal conductivity according to claim 1, wherein in step S6, calculating the ductile-to-brittle transition temperature increment ΔT of the metal sample to be tested after irradiation by equation (2): $ΔT = 178.1 \times \left({H}_{p}-{H}_{0}\right)$ (2) wherein, Hₚ represents the nanohardness of the metal sample to be tested after irradiation, and H₀ represents the nanohardness of the unirradiated metal sample.
